# EUROPEAN PATENT APPLICATION

(11) **EP 4 534 157 A1**
(43) Date of publication of application: **09.04.2025**
(21) Application number: 23815351.4
(22) Date of filing: 25.05.2023
(51) Int. Cl.: A63B 69/00

(54) **TRAINING SYSTEM FOR ATHLETES OF MARTIAL ARTS AND CONTACT SPORTS**

(30) Priority: 31.05.2022 ES 202230908 U
(71) Applicant: Toscano Martin, Didac, 08784 Barcelona (ES); Campos Fernandez, Francisco Javier, 08784 Barcelona (ES)
(72) Inventor: Toscano Martin, Didac, 08784 Barcelona (ES); Campos Fernandez, Francisco Javier, 08784 Barcelona (ES)
(74) Representative: Arsuaga Santos, Elisa
(86) International application number: PCT/ES2023/070339
(87) International publication number: WO 2023/233053

(57) **Abstract**

The present invention relates to a training system for martial arts and contact sports that has three basic groups for the proper functioning thereof. The first group consists of a series of sensors called limbs, since they are coupled to each of the four limbs of the individual who is training. The second group consists of a series of sensors that is called the ring or training area. The third group consists of two analytics applications with two functional levels, the data of the individual who is training and comparative data from groups of individuals.

## Description

### FIELD OF INDUSTRIAL APPLICATION

The invention relates to a technological solution consisting of a group of electronic devices that are designed, manufactured, and calibrated expressly for the detection of critical parameters in training processes and martial arts combats, such as karate, taekwondo, Muay Thai, as well as other contact sports, such as boxing, MMA, K1, etc.

### PRIOR ART

The world of sport has evolved substantially in terms of technology in recent years. The inclusion of electronic devices and the communication between them has made it possible to study data and obtain precise information which, in turn, has had a very significant impact on the processes of improvement in sport.

The invention, as described above, lies in the linking of a group of measurement devices designed and manufactured to improve training systems, for martial arts and contact sports athletes, specifically Muay Thai, which provide substantial advantages over the techniques known to date.

There are several prior art documents that claim some aspects of this invention, most of which measure impacts on the punching bag. None of them, however, covers a system as complete as the one in this utility model application.

US patent US20220079471 relates to a system for monitoring the physiological aspects of an individual during physical exercise, displaying an alarm when certain pre-set limits are exceeded.

Romanian patent RO134344 describes a fitness data acquisition kit, which consists of mass measurement sensors, a movement measurement sensor/proximity sensor, and a module the function of which is to collect and process the information from the sensors and transmit same.

EP3106211 is a European patent filed in March 2008 and granted in November 2019, but was not validated in Spain, so it does not come into effect in this country. It relates to a complete article of clothing.

EP0222640 is a European patent filed in October 1986, but was withdrawn in September 1990, so it has not come into effect in Spain either. The sensors it has are included in the object of impact, such as the punching bag, etc., and not on the individual.

US patent US2017291086 comprises three training exercise control phases.

As for Spanish utility model ES1210713, it is based on a training frame, bags that are called impact receivers, the projection of videos with music on a display, Wi-Fi sensors with an accelerometer, and several software components for control and scoring. It is aimed at group performances.

### DESCRIPTION OF THE INVENTION

The training system for martial arts and contact sports has three basic groups for the proper functioning thereof.

The first group consists of a series of sensors called limbs, since they are coupled to each of the four limbs of the individual who is training, although there could be fewer in number.

The second group consists of a series of sensors that is called the ring or training area. Generally there are three, although the number of these sensors may vary.

The third group consists of two analytics applications with two functional levels, the data of the individual who is training and comparative data from groups of individuals.

### DETAILED DESCRIPTION OF AN EMBODIMENT

There are three types of sensors included in the system, which are: upper limb sensors, lower limb sensors, and training area or locating sensors. Two real-time applications are included for the processing of all the data generated.

**The limb sensors** include an electronic device that can be coupled to each of the limbs of the analyzed athlete by means of a fabric case. This device has its own electronics board, capable of transmitting information in real time to the mentioned mobile application, which has been developed exclusively to interpret and display the training data in real time.

The electronic devices of the sensors coupled on the limbs communicate with one another through a local network protocol, which allows the coordination between same, as well as the transmission of data in a coherent manner to the mobile application.

The sensors include an aid system capable of guiding the user to place the devices at the proper points of the limbs. They are also capable of self-calibrating in order to distinguish the movements that are blows from those that are not.

These electronic devices include a pulse measurement sensor.

Using a printed circuit custom-designed for the **sensors positioned on the upper limbs,** blows in the different martial arts and contact sports are identified without the need to include receiving elements that accompany the identification and classification of blows.

Straight and curved blows executed with these limbs are identified, and in particular the types of blows associated with each of the martial arts and contact sports, for example, in the case of Muay Thai:
Jab, cross or direct, upward hook, crochet and elbows.

Although the sensor is located on the wrist, due to the paths of execution, blows are detected and counted by standard accelerations, decelerations at specific points, and turns necessary for the proper projection thereof.

In addition to counting these blows, the sensor itself defines what is a blow and what is not, and determines the force and speed of execution.

The upper sensors are furthermore responsible for combining the physiological data of the individual, this being a key element in the development of algorithms to determine the athlete's sports performance during the warm-up, training, and recovery process.

The sensors are located on the wrists and are numbered, as each one contains a specific configuration for the proper functioning thereof.

**The lower limb sensors** are located in the back side and slightly above the lower joints of both legs.

They are fixed by means of a piece of fabric that allows them to be adjusted, this being a secure and at the same time ergonomic fixing.

Their main functions lie in the detection of leg blows (kicks) that are executed with both limbs, counting the number of executions per workout, as well as the force of execution in each case.

Similarly, the sensors, although located at the lower joints, are capable of detecting blows that are executed with the knees, regardless of their form of execution: frontal, lateral, with jumps, circular and diagonal.

In the case of its use in Muay Thai, boxing, MMA, etc., the most common detections are, among others, frontal kicks and circular kicks.

The **locating sensors** consist of electronic elements, which function autonomously by means of batteries, and they can also be powered by the power grid. At least three sensors are positioned in the fight area.

Once suitably positioned according to the algorithm of intensities and distances, a wireless communication protocol is established between the mobile applications of the players involved in the fight, the four devices located on the limbs of each player, and the locating sensors.

Based on the variations in intensity between the four data collection sensors, located on the limbs of each individual, the exact locations of each athlete involved in the fight area are identified.

This locating function in turn allows the transmission of data to the mobile application, where said variations are collected and processed to represent in a graph the fight perimeter, the different locations adopted by the fighter or individuals involved. Said variations are represented through a heat map which, with colors described in a legend, allows the movements of the fighter to be analyzed regardless of the execution planes and speeds.

This function shared between all sensors allows the measurement of the following, among others:
Mean distance between fighters within the fight area.

Distance covered by each of the fighters.

Definition of fight areas where each athlete involved feels more comfortable executing blows, a combination that takes into account the area where greater and better executions are produced based on force.

Mean speed of movements within the fight area.

**Mobile applications** are available for IOS and Android environments. They collect in real time the data sent by the electronic devices integrated on the athlete(s) and in the training area. With all this, the management of users within each of the environments, as well as the interaction between them, is performed, thereby allowing comparisons of objective data. The mobile application will allow the memorization or storage of data, thus allowing the interpretation and reinterpretation of behaviors during training processes and improving diagnostic systems and training programs.

There are two types of applications for this system: one for the **management of events** developed in each session and another one for the **management of administrators and users of the system.**

**The application for management of events** is capable of displaying in real time on a display the basic information of the performance of the individual who has been training, or display the comparison of the performance of two athletes performing at the same time. In the latter case, there will be eight devices, four for each of them.

The data that are displayed and can be compared are:
▪ Number of curved blows per limb with a sensor.
▪ Number of straight blows per limb with a sensor.
▪ Force of the projected blows.
▪ Speed of the projected blows.
▪ Strikes by each of the players.
▪ Current round number.
▪ Strongest blow recorded by each of the players.
▪ Total time of the round with countdown.

These can be used to create both an audible and visual animation.

Specific tracks can also be reproduced on the display when some of the most important events occur, such as strongest blow recorded by each player, strongest blow recorded in the fight, player's highest strikes during the fight, difference of more than 20 blows executed by a player, weakest blow recorded by the player, or weakest blow recorded in the fight.

**The application for management of administrators** and users allows:
- Management of administrators and users of the system and their corresponding databases.
- Establish communication protocols between the different components.
- Mailing systems.
- Control of the use of the corresponding social network.
- Advertising management.
- Autonomous user registration.
- Environment for users in training mode with hidden or displayable data.
- Comparison of results, in graphical or tabular mode.
- Any user can receive various notifications.
- Possibility of challenges between users.
- Possibility of gym registration.
- Possibility of guest registration.
- Depending on the data received, the application can identify the areas of greater and lesser permanence of the athlete or of a greater or lesser number of blows executed, representing them with heat maps using different colors.

## Claims

1. A training system for athletes of martial arts and contact sports aimed at individual use and group use, which includes a series of sensors and an application developed for systems of this type, **characterized in that** it comprises:
- four electronic limb devices based on sensors, two in the upper limbs (arms) and two in the lower limbs (legs) of the person who is training and which are suitable for self-calibrating;
- several locating sensors, at least three in number, which function autonomously by means of batteries or which are powered by the power grid;
- an electronic device including at least one mobile application, configured to communicate wirelessly with each of the four devices placed on the person who is training, and with the locating sensors.

2. The training system for athletes of martial arts and contact sports according to claim 1, **characterized in that** the arm sensors are positioned on the wrists and the leg sensors are positioned on the back side, above the lower leg joints, all of them fastened with a piece of fabric that allows them to be adjusted.

3. The training system for athletes of martial arts and contact sports according to claim 1, **characterized in that** the device has its own electronics board, battery, and pulse measurement sensors configured to use a printed circuit and designed to communicate with one another through a local network protocol.

4. The training system for athletes of martial arts and contact sports according to claim 1, **characterized in that** the limb sensors are designed to combine the physiological data of the individual and are located on the board.

5. The training system for athletes of martial arts and contact sports according to claim 1, **characterized in that** the sensors include an aid system configured to guide the user for placing the devices at the proper points of the limbs.

## Amended claims

### Amended claims under Art. 19.1 PCT

1. A training system for athletes of martial arts and contact sports aimed at individual use and group use, which includes a series of sensors and an application developed for systems of this type, **characterized in that** it comprises:
- four electronic limb devices based on sensors, two in the upper limbs (arms) and two in the lower limbs (legs) of the person who is training and which are suitable for self-calibrating;
- several locating sensors, at least three in number, which function autonomously by means of batteries or which are powered by the power grid;
- an electronic device including at least one mobile application, configured to communicate wirelessly with each of the four devices placed on the person who is training, and with the locating sensors.

2. The training system for athletes of martial arts and contact sports according to claim 1, **characterized in that** the arm sensors are positioned on the wrists and the leg sensors are positioned on the back side, above the lower leg joints, all of them fastened with a piece of fabric that allows them to be adjusted.

3. The training system for athletes of martial arts and contact sports according to claim 2, wherein the piece of fabric is a case adapted specifically for the corresponding limb of the user.

4. The training system for athletes of martial arts and contact sports according to any of the preceding claims, **characterized in that** the device has its own electronics board, battery, and pulse measurement sensors configured to use a printed circuit and designed to communicate with one another through a local network protocol.

5. The training system for athletes of martial arts and contact sports according to any of the preceding claims, **characterized in that** the limb sensors are designed to combine the physiological data of the individual and are located on the board.

6. The training system for athletes of martial arts and contact sports according to any of the preceding claims, **characterized in that** the sensors include an aid system configured to guide the user for placing the devices at the proper points of the limbs

7. The training system for athletes of martial arts and contact sports according to any of the preceding claims, wherein each of the locating sensors is arranged to receive respective locating signals from all the limb sensors of an individual, such that the combination of said locating signals allows each individual in the fight area to be located accurately.
